# EUROPEAN PATENT SPECIFICATION

(11) **EP 2 671 552 B1**
(45) Date of publication and mention of the grant of the patent: **14.09.2016**
(21) Application number: 12741628.7
(22) Date of filing: 01.02.2012
(51) Int. Cl.: A61F 13/49, A61F 13/514, A61F 13/51, A61F 13/515, A61F 13/539, A61F 13/15

(54) **ABSORBENT ARTICLE**
SAUGFÄHIGER ARTIKEL
ARTICLE ABSORBANT

(30) Priority: 01.02.2011 JP 2011020259
(43) Date of publication of application: 11.12.2013
(73) Proprietor: Unicharm Corporation, Ehime 799-0111 (JP)
(72) Inventor: NAGAI, Takahito, Kanonji-shi Kagawa 769-1602 (JP); TSUJI, Tomoko, Kanonji-shi Kagawa 769-1602 (JP); TAGAWA, Nobuhiro, Kanonji-shi Kagawa 769-1602 (JP)
(74) Representative: Dolleymores
(86) International application number: PCT/JP2012/052214
(87) International publication number: WO 2012/105589

(56) References cited:
- EP-A2- 1 078 620
- WO-A1-99/14415
- JP-A- 2001 008 969
- JP-A- 2001 046 426
- JP-A- 2006 130 042
- JP-A- 2009 056 142
- JP-A- 2010 279 725
- US-A1- 2003 120 245
- US-A1- 2005 137 549
- US-A1- 2008 228 158

## Description

### [Technical Field]

The present invention relates to an absorbent article such as an open-type disposable diaper using a fastening tape.

### [Background Art]

Conventionally, there is known an open-type disposable diaper using a fastening tape. Such a disposable diaper often uses, as the fastening tape, a structure in which a male member of a mechanical fastener is provided on a side panel extending from the dorsal region of the disposable diaper. Furthermore, in the ventral region of such a disposable diaper, generally, a target tape that engages with the male member of the mechanical fastener is provided.

For such a disposable diaper, there is proposed a disposable diaper in which rather than using the target tape, that is, the female member of the mechanical fastener, the male member of the mechanical fastener is directly engaged with an outer-layer nonwoven fabric arranged outside an barrier sheet of the disposable diaper (for example, Patent Literature 1). In the disposable diaper, in order to ensure a perfect engagement of the male member with the outer-layer nonwoven fabric, as well as the strength of the outer-layer nonwoven fabric, adhesive-applied units of the barrier sheet and the outer-layer nonwoven fabric are provided linearly at a predetermined interval.

### [Citation List]

### [Patent Literature]

[Patent Literature 1]
Japanese Patent Application Publication No. 2001-46426 (Page 3, Fig. 2)
A further prior art arrangement is known from US2008/0228158.

### [Summary of Invention]

However, the aforementioned conventional disposable diaper has the problems below. That is, the adhesive-applied units of the barrier sheet and the outer-layer nonwoven fabric are linearly provided, and therefore, the adhesive easily seeps out to the surface of the outer-layer nonwoven fabric. The adhesive that seeps out restricts the free movement of the fibers forming the outer-layer nonwoven fabric, and thus, the engagement force of the male member of the mechanical fastener with the outer-layer nonwoven fabric declines.

Furthermore, in a portion of the outer-layer nonwoven fabric that is not bonded with the barrier sheet, the repeated use of the fastening tape stretches and keeps undoing the fibers engaged with the male member, and as a result, the outer surface of the outer-layer nonwoven fabric is easily covered. This further declines the engagement force of the male member with the outer-layer nonwoven fabric.

Thus, the present invention has been achieved in view of such a situation, and an object thereof is to provide an absorbent article, such as a disposable diaper, by which it is possible to maintain the engagement force of the fastening tape even after repeated use while ensuring the strength of an outer-layer nonwoven fabric, in a case of using a structure in which rather than making use of a target tape, the fastening tape is engaged directly with the outer-layer nonwoven fabric.

According to the present invention in an aspect, there is provided an absorbent article as recited by claim 1.

### [Brief Description of Drawings]

[Fig. 1] Fig. 1 is an exploded plan view of a disposable diaper 1 according to an embodiment of the present invention.
[Fig. 2] Fig. 2 is a diagram schematically illustrating a cross section of the disposable diaper 1 along F2-F2 shown in Fig. 1.
[Fig. 3] Fig. 3 is a diagram illustrating an application pattern of an adhesive in an adhesive layer 80 according to an embodiment of the present invention.
[Fig. 4] Fig. 4 is a diagram illustrating an application pattern of an adhesive in an additional adhesive layer 90 according to an embodiment of the present invention.

### [Description of Embodiments]

Next, an embodiment of an absorbent article according to the present invention is explained with reference to drawings. In the following description of the drawings, the same or similar reference numerals are used to designate the same or similar parts. It will be appreciated that the drawings are schematically shown and the ratio of each dimension, for example, are different from the real ones.

Therefore, a specific dimension should be determined in view of the following description. Moreover, among the drawings, the respective dimensional relations or ratios may differ.

### (1) Overall Schematic Configuration of Absorbent Article

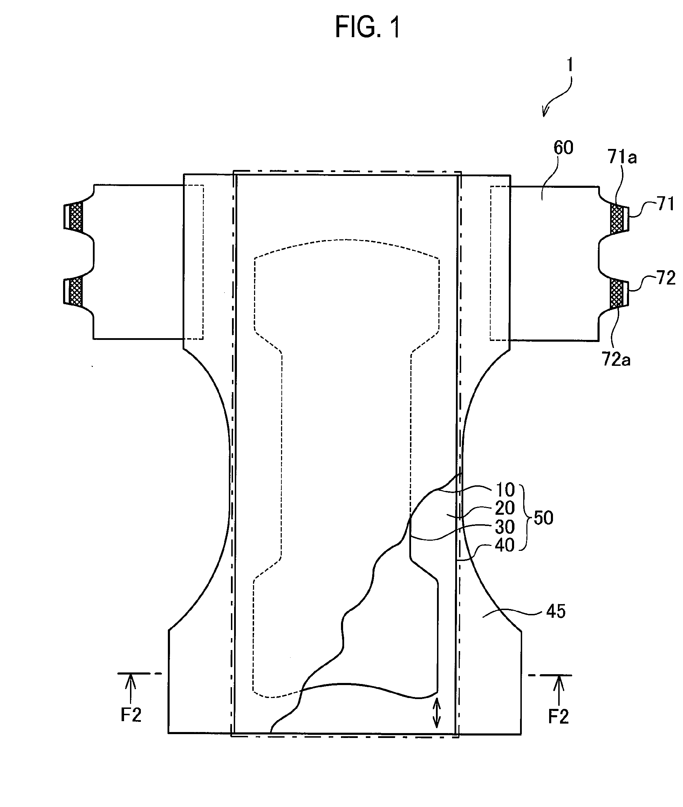

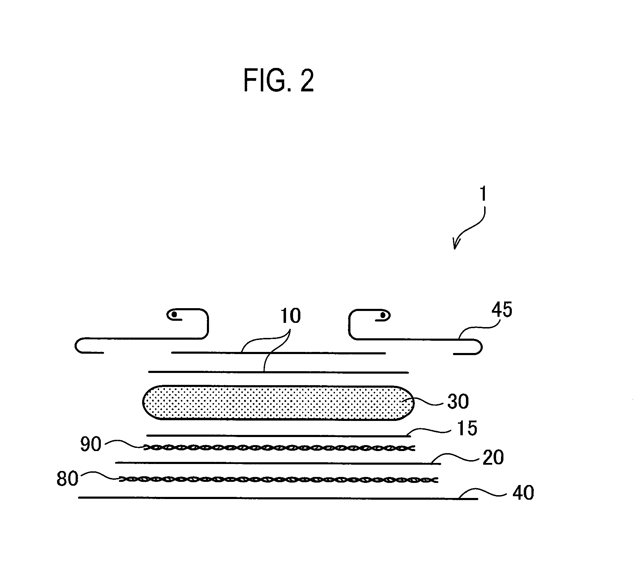

Fig. 1 is an exploded plan view of a disposable diaper 1, which is an embodiment of the absorbent article according to the present invention. Fig. 2 is a diagram schematically illustrating a cross section of the disposable diaper 1 along F2-F2 shown in Fig. 1.

As illustrated in Fig. 1 and Fig. 2, the disposable diaper 1 is provided with a topsheet 10, a barrier sheet 20, an absorber 30, and an outer-layer nonwoven fabric 40, and includes a substantially longitudinally-elongated absorbent chassis 50.

The topsheet 10 is a liquid-permeable sheet that is in direct contact with the skin of a wearer. The topsheet 10, for example, is formed by a sheet made of hydrophilic nonwoven fabric, an aperture plastic film, or an aperture hydrophobic nonwoven fabric.

The barrier sheet 20 is a liquid-impermeable sheet that prevents the liquid absorbed in the absorber 30 from leaking to the outside. The barrier sheet 20, for example, is formed by a polyethylene (PE) film sheet.

The absorber 30, which has a longitudinally elongated shape, has liquid retention property that the liquid excreted from the wearer is absorbed. The absorber 30, for example, is formed by a material obtained by mixing cotton-like pulp and a highly polymerized absorbent polymer (SAP). The absorber 30 is provided between the topsheet 10 and the barrier sheet 20.

It should be noted that in the present embodiment, a lower-layer sheet 15 (see Fig. 2) is further provided between the barrier sheet 20 and the absorber 30. The lower-layer sheet 15 is a sheet narrower in width than the barrier sheet 20. The lower-layer sheet 15, for example, is formed by a tissue or a hydrophilic nonwoven fabric sheet.

The outer-layer nonwoven fabric 40 is provided outside the barrier sheet 20. The material used as the outer-layer nonwoven fabric 40 is not particularly limited as long as fastening tapes 71, 72 provided in side panels 60 can be engaged; however, examples of preferred constituent fibers include fibers that consists only of polyolefin fibers such as polyethylene (PE) fibers and polypropylene (PP) fibers, and thermoplastic resins such as polyethylene terephthalate (PET) and polyamide. Another example thereof may include composite fibers having a core-sheath type or a side-by-side type structure, that is, fibers having a core-sheath structure in which the core component is a PE or a low-melting point PP. In addition, the outer-layer nonwoven fabric 40 may be composed by using these fibers either individually or in a combination of two or more types.

On both sides of the outer-layer nonwoven fabric 40, a leg side gather (LSG) nonwoven fabric 45 having elastic members such as elongated synthetic rubber arranged along the longitudinal direction of the absorbent chassis 50 is provided.

A pair of left-right side panels 60 extending from the sides of the absorbent chassis 50 are bonded between the outer-layer nonwoven fabric 40 and an LSG nonwoven fabric 45. The side panels 60 are formed to be capable of expanding and contrasting in a shorter direction perpendicular to the longitudinal direction of the absorbent chassis 50. A well-known method can be used as a method of providing such flexibility to the side panels 60, and examples thereof may include a method of bonding a plurality of elastic members that are elongated in the shorter direction onto a nonwoven fabric forming the side panels 60.

The fastening tape 71 and the fastening tape 72 are provided at the sides of a pair of left-right side panels 60. A male member 71a of the mechanical fastener is provided on the fastening tape 71. Similarly, a male member 72a of the mechanical fastener is provided on the fastening tape 72.

The pair of left-right side panels 60 are provided in a dorsal region of the absorbent chassis 50 positioned at the dorsal side of the wearer. In the disposable diaper 1, a target tape (a female member of the mechanical fastener) where the male member 71a and the male member 72a are engaged is not provided. That is, the male member 71a and the male member 72a are engaged directly with the surface of the outer-layer nonwoven fabric 40. Specifically, the male member 71a and the male member 72a can be engaged to any region (within a frame indicated by a dashed line in Fig. 1) on the surface of the outer-layer nonwoven fabric 40.

It should be noted that such a structure of the disposable diaper 1 is same as that of a well-known open-type disposable diaper.

The present embodiment is designed such that in a state where the side panels 60 are elongated to the maximum extent and the male member 71a and the male member 72a are engaged with the outer-layer nonwoven fabric 40, the stress exerted on the side panels 60 is 4.0 N or less. Furthermore, two, not one, male members are provided on each of the side panels 60, and therefore, the stress is distributed by the elastic members of the side panels 60 so as to achieve a feature that the stress obtained when the side panels 60 are elongated to the maximum extent is 4.0 N or less. A method of measuring the stress will be described later.

Furthermore, as illustrated in Fig. 2, between the barrier sheet 20 and the outer-layer nonwoven fabric 40, an adhesive layer 80 formed by an adhesive that bonds the barrier sheet 20 and the outer-layer nonwoven fabric 40 is provided. The adhesive layer 80 bonds the barrier sheet 20 and the outer-layer nonwoven fabric 40.

Moreover, in the present embodiment, between the barrier sheet 20 and the lower-layer sheet 15, an additional adhesive layer 90 formed by an adhesive that bonds the barrier sheet 20 and the lower-layer sheet 15 is provided. The additional adhesive layer 90 bonds the barrier sheet 20 and the lower-layer sheet 15.

That is, the adhesive layer 80 and the additional adhesive layer 90 result in the formation of the outer-layer nonwoven fabric 40, the barrier sheet 20, and the lower-layer sheet 15 as one unit.

### (2) Application pattern of adhesive

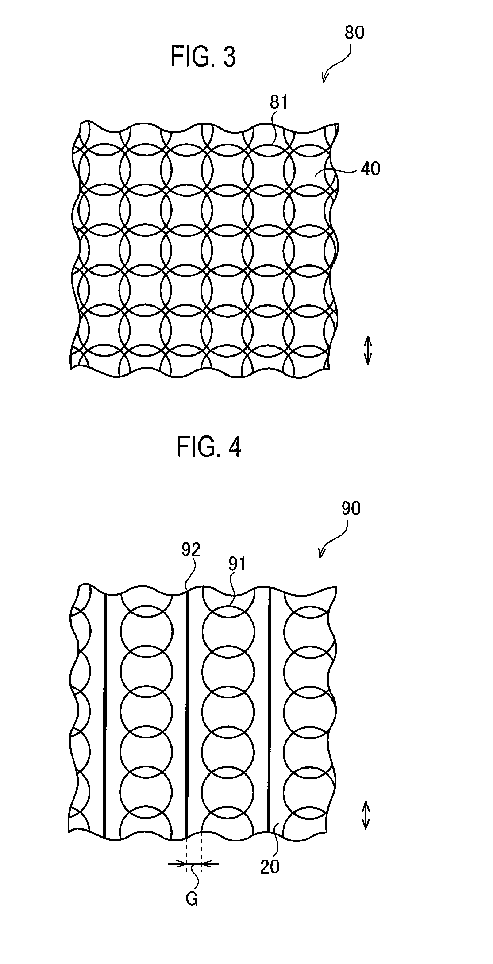

Next, an application pattern of the adhesive in the above-described adhesive layer 80 and the additional adhesive layer 90 will be described. Fig. 3 illustrates an application pattern of the adhesive in the adhesive layer 80. Fig. 4 illustrates an application pattern of the adhesive in the additional adhesive layer 90.

### (2.1) Adhesive layer 80

As shown in Fig. 3, in the adhesive layer 80, the application pattern of an adhesive 81 has spiral shape. The adhesive 81 forms an application pattern in which the spiral shape is repeated along the longitudinal direction of the absorbent chassis 50.

Furthermore, such an application pattern crosses an orientation (the direction of an arrow in Fig. 1 and Fig. 3) of the fibers forming the outer-layer nonwoven fabric 40 and an orientation of the resin forming the barrier sheet 20. The orientation of the fibers forming the outer-layer nonwoven fabric 40 means a direction in which the fibers forming the outer-layer nonwoven fabric 40 extend, and more specifically, means a direction in which a majority of fibers (including the resin) forming the outer-layer nonwoven fabric 40 extend. Furthermore, the orientation of the resin forming the barrier sheet 20 also carries the same meaning as the outer-layer nonwoven fabric 40. A method of measuring the orientation of fibers and the resin will be described later.

The adhesive 81 is applied so as to cover the entire surface of the barrier sheet 20, and the barrier sheet 20 and the outer-layer nonwoven fabric 40 are fixed by using a surface that is broader than a line and a point. More specifically, the adhesive 81 is applied on the entire surface of the barrier sheet 20 to form a net-like or mesh-like shape, the barrier sheet 20 and the outer-layer nonwoven fabric 40 are bonded and fixed uniformly, and at the same time, the fibers of the outer-layer nonwoven fabric 40 in a non-bonded region have a proper degree of freedom.

Examples of the adhesive 81 include a rubber-type hot-melt adhesive in which a styrene elastomer and EVA are used as a base polymer, and a hot-melt adhesive in which an amorphous poly-alpha olefin is used as a base polymer. Furthermore, the basis weight of the adhesive 81 forming the adhesive layer 80 is preferably 1.0 to 10 g/m². If the basis weight is less than 1.0 g/m², the fiber damage becomes remarkable at the time of engagement and disengagement of the fastening tapes 71 and 72, and therefore, it is not preferable. On the other hand, if the basis weight exceeds 10 g/m², the adhesive 81 restricts the degree of freedom of the fibers due to which the disengagement strength declines, and therefore, it is not preferable.

In addition, the bonding strength of the adhesive layer 80 is preferably between 1.0 and 10 N/25 mm. If the bonding strength is less than 1.0 N/25 mm, the outer-layer nonwoven fabric 40 is pulled by the shearing force of the male members 71a, 72 (hook), and the outer-layer nonwoven fabric 40 and the barrier sheet 20 may peel off. On the other hand, if the bonding strength exceeds 10 N/25 mm, then when the outer-layer nonwoven fabric 40 is pulled by the shearing force of the male members 71a, 72 (hook), the relief of stress (expanding and contrasting of fibers) is not possible, and therefore, the stress is transferred to the barrier sheet 20 due to which the barrier sheet 20 elongates, and perforations, for example, occur easily. A method of measuring the bonding strength will be described later.

It should be noted that the application pattern of the adhesive 81 is not limited to the spiral shape as shown in Fig. 3. Specifically, any application pattern may be acceptable such as that which is formed by coating in a spiral-shaped pattern, coating in an omega (Ω)-shaped pattern, coating in a wave-shaped pattern, or coating in a sheet-shaped pattern, as long as the application pattern crosses the orientation of the fibers forming the outer-layer nonwoven fabric 40, and is also continuous in the longitudinal direction and the shorter direction of the absorbent chassis 50. Coating in a sheet-shaped pattern is a pattern in which a fine thread-like hot-melt adhesive resin is applied on the entire surface of a region to be bonded to result in a sheet-like state. Even in such a case, the application pattern of the adhesive resin is to include an element elongated in the direction that crosses the orientation of the fibers (the orientation of the resin) of the outer-layer nonwoven fabric 40 (the barrier sheet 20), and the element constituting the application pattern is to cross in a mesh-like shape.

That is, the application pattern of the adhesive 81 may be acceptable as long as it is shaped in the form of a net or a mesh, and as a result of such an application pattern, the barrier sheet 20 and the outer-layer nonwoven fabric 40 are fixed uniformly, and at the same time, the fibers of the outer-layer nonwoven fabric 40 positioned in the unbonded region between the fibers have the proper degree of freedom, hence preferable. On the other hand, in a case of coater coating (applying the adhesive thinly over a surface), the adhesive is applied on the entire surface of the outer-layer nonwoven fabric 40, and thus, the degree of freedom of the outer-layer nonwoven fabric 40 is restricted, resulting in a decline in the disengagement strength, hence not preferable.

### (2.2) Additional adhesive layer 90

As illustrated in Fig. 4, in the additional adhesive layer 90, the application pattern of an adhesive 91 has spiral shape similarly to the adhesive 81. The adhesive 91 forms an application pattern in which the spiral shape is repeated along the longitudinal direction of the absorbent chassis 50. Furthermore, in the additional adhesive layer 90, the application pattern of an adhesive 92 stretches along the longitudinal direction of the absorbent chassis 50, and specifically, has an approximately parallel linear shape with respect to the longitudinal direction of the absorbent chassis 50.

That is, the additional adhesive layer 90 includes a portion (a first portion) having an application pattern of the adhesive 91 that crosses the orientation (the direction of an arrow shown in Fig. 4) of the resin configuring the barrier sheet 20, and a portion (a second portion) having an application pattern of the adhesive 92 that is approximately parallel to the orientation of the resin.

Furthermore, in the shorter direction that is perpendicular to the longitudinal direction of the absorbent chassis 50, an interval G between the portion (the first portion) having the application pattern of the adhesive 91 and the portion (the second portion) having the application pattern of the adhesive 92 is preferably 20 mm or less. A hot-melt adhesive similar to the adhesive 81 can be used as the adhesive 91 and the adhesive 92.

Similarly to the adhesive 81, the application pattern of the adhesive 91 may be accepted as long as it crosses the orientation of the resin forming the barrier sheet 20, and at the same time, continues in the longitudinal direction and the shorter direction of the absorbent chassis 50. Furthermore, the application pattern of the adhesive 92, for example, can be formed by bead coating.

The basis weight of the adhesive 91 and 92 forming the additional adhesive layer 90 can be configured similarly to that of the adhesive 81. Furthermore, a pasting WET strength of the additional adhesive layer 90 is preferably 0.3 N/25 mm or more. If the pasting WET strength is less than 0.3 N/25 mm, the strength is not sufficient so that the barrier sheet 20, for example, peel off at the time of using the disposable diaper 1.

### (3) Method of measuring the orientation of the fibers (resin) of the outer-layer nonwoven fabric 40 (the barrier sheet 20)

It is possible to determine the orientation of the fibers of the outer-layer nonwoven fabric 40 and the orientation of the resin of the barrier sheet 20 on the basis of the tensile strength of the sheet (nonwoven fabric) made of thread-like fibers and the sheet (film) made of resin. Specifically, it is possible to measure the orientation of the fibers or the resin can be measured according to a procedure described below.
- Create a sample of a sheet (the outer-layer nonwoven fabric 40 or the barrier sheet 20) to be measured that extends along the lengthwise direction and the crosswise direction of the product.
- Measure the tensile strength at the time of elongation of the sample by a predetermined amount by using a tensile strength tester.

For the tensile strength test, a tensile strength tester manufactured by Instron Japan Co., Ltd. (for example, model: 5564) was used. A sample having a size (10-mm width and 100-mm length) matching a sample gripping portion of the tester is pulled at a tension rate of 100 mm/min, and the tensile strength is measured when the sample is distorted by 10% with respect to the initial length. In this case, when the tensile strength of the sample extending along a lengthwise direction Y is greater than that of the sample extending along a crosswise direction X, it is possible to determine that the fibers (resin) are oriented along the lengthwise direction Y

### (4) Method of measuring stress of the side panels 60

It is possible to measure the stress at the time of maximum elongation of the side panels 60 according to a procedure described below.
- Separate the side panels 60, including the portion bonded with the absorbent chassis 50, from the absorbent chassis 50.
- Cut out the separated side panels 60 at a width of 50 mm, and measure a load (tensile strength) at the time of elongation of the side panels 60 in the expanding and contrasting direction (the shorter direction of the absorbent chassis 50, specifically, the widthwise direction of the disposable diaper 1) by using a tensile strength tester.

For the tensile test, a tensile strength tester manufactured by Instron Japan Co., Ltd. (for example, model: 5564) was used. At this time, fix the bonded ends of the side panels 60 and the absorbent chassis 50, as well as the male member 71a or the male member 72a to the chuck. Following this, the load at the time of maximum elongation of the side panels 60 was measured at a tension rate of 300 mm/min.

### (5) Method of measuring the bonding strength

It is possible to measure the bonding strength between the outer-layer nonwoven fabric 40 and the barrier sheet 20, and that between the barrier sheet 20 and the lower-layer sheet 15 according to a procedure described below.
- From the region of the absorbent chassis 50 where the fastening tapes 71 and 72 are affixed, cut out a sample of a size of 25-mm width × 100-mm length.
- Sandwich the sample by a measurement chuck (at top and bottom two places), move the upper measurement chuck at a direction of 180° with respect to the lower measurement chuck (in a vertical upward direction) at a speed of 100 mm/min, and measure the maximum speed (N) at that time. For the bonding strength test, a tensile strength tester manufactured by Instron Japan Co., Ltd. (for example, model: 5564) was used. It should be noted that the pasting WET strength of the additional adhesive layer 90 was measured by using a similar method.

As described above, in the disposable diaper 1, the barrier sheet 20 and the outer-layer nonwoven fabric 40 are fixed over the surface by the adhesive layer 80. The adhesive layer 80 has an application pattern that crosses the orientation of the fibers forming the outer-layer nonwoven fabric 40 and the orientation of the resin forming the barrier sheet 20, specifically, the adhesive layer 80 has an application pattern in which the spiral shape is repeated along the longitudinal direction of the absorbent chassis 50.

In such disposable diaper 1, even when the male members 71a, 72a of the fastening tapes 71 and 72 are engaged with the outer-layer nonwoven fabric 40 and the fastening tapes 71, 72 are pulled up, a completely opposite force (retaining force) by which the outer-layer nonwoven fabric 40 is kept remaining at the same position is generated against the force for pulling up the fastening tapes 71, 72.

Therefore, the retaining force of the entire region of the outer-layer nonwoven fabric 40 allows for reduction of stretching and undoing of the fibers of the unbonded outer-layer nonwoven fabric 40. That is, it is possible to restrain the detaching of the fibers of the outer-layer nonwoven fabric 40 engaged with the male members 71a, 72a from the male members 71a, 72a before the elongation due to disengagement, and the tearing of the fibers entwined with the male members 71a, 72a. Furthermore, because the application pattern of the adhesive 81 is such that the spiral shape is repeated along the longitudinal direction of the absorbent chassis 50, the adhesive 81 hardly seeps out to the surface of the outer-layer nonwoven fabric.

That is, according to the disposable diaper 1, when using a structure in which rather than making use of a target tape, the fastening tapes 71, 72 are directly engaged with the outer-layer nonwoven fabric 40, it is possible to maintain the engagement force of the fastening tapes 71, 72 even after repeated use while ensuring the strength of the outer-layer nonwoven fabric 40.

In addition, in the present embodiment, the barrier sheet 20 and the lower-layer sheet 15 are bonded by the additional adhesive layer 90. The additional adhesive layer 90 includes a portion having an application pattern of the adhesive 91 that crosses the orientation of the resin configuring the barrier sheet 20, and a portion having an application pattern of the adhesive 92 that is approximately parallel to the orientation of the resin configuring the barrier sheet 20.

Therefore, the outer-layer nonwoven fabric 40, the barrier sheet 20, and the lower-layer sheet 15 are formed as one unit, and the elongation of the barrier sheet 20 is restrained even when a shearing stress is exerted on the outer-layer nonwoven fabric 40, the barrier sheet 20, and the lower-layer sheet 15. When the elongation of the barrier sheet 20 is restrained, the barrier sheets 20 are hardly bonded to each other, and the joining and twisting between the barrier sheets 20, as well as the perforations in the barrier sheet 20 due to such joining and twisting are restrained.

Furthermore, the application pattern of the adhesive 92 is a straight line by bead coating, and thus, the additional adhesive layer 90 can withstand the movement of the wearer even when the additional adhesive layer 90 becomes wet by a bodily waste of the wearer, and the bonding strength by which the barrier sheet 20 and the lower-layer sheet 15 do not peel off can be easily ensured. It should be noted that when the application pattern of the adhesive is non-continuous such as curtain coating, moisture easily enters in between the adhesive, making it difficult to increase the bonding strength.

In addition, in the present embodiment, the interval between the portion having the application pattern of the adhesive 91 and the portion having the application pattern of the adhesive 92 is set to 20 mm or less. Therefore, the additional adhesive layer 90 can withstand the movement of the wearer even when the additional adhesive layer 90 becomes wet by the bodily waste of the wearer, and the bonding strength by which the barrier sheet 20 and the lower-layer sheet 15 do not peel off can be easily ensured.

The present embodiment is designed such that in a state where the side panels 60 are elongated to the maximum extent and the male member 71a and the male member 72a are engaged with the outer-layer nonwoven fabric 40, the stress exerted on the side panels 60 is 4.0 N or less. Therefore, a load caused by the shearing force is hardly exerted on the outer-layer nonwoven fabric 40, which is a region in which the male member 71a and the male member 72a are engaged. In other words, the damage to the outer-layer nonwoven fabric 40 caused by the repeated use of the disposable diaper 1 can be retarded.

In addition, the male member 71a and the male member 72a can be engaged to any region (within a frame indicated by a dashed line in Fig. 1) on the surface of the outer-layer nonwoven fabric 40, and because the side panels 60 have flexibility, the disposable diaper 1 can comply with the most appropriate style of wearing in accordance with various body types and physical conditions of the wearer, particularly elderly people.

### (6) Other embodiments

As described above, the present invention is disclosed through the embodiments of the present invention. However, it should not be interpreted that the statements and drawings constituting a part of the present disclosure limit the present invention. From this disclosure, a variety of alternate embodiments, examples, and applicable techniques will become apparent to one skilled in the art.

For example, in the above-described embodiment, the side panels 60 had flexibility; however, the side panels 60 need not necessarily have flexibility.

Furthermore, in the above-described embodiment, the barrier sheet 20 and the lower-layer sheet 15 were bonded by the additional adhesive layer 90, however, the barrier sheet 20 and the lower-layer sheet 15 need not necessarily be bonded by the additional adhesive layer 90.

In addition, in the above-described embodiment, the explanation was provided with the disposable diaper 1 as an example, however, the absorbent article to which the present invention can be applied is not limited to a disposable diaper, and can be widely applied in an absorbent article having a male member of a mechanical fastener provided at the sides of the side panels.

As described above, needless to say, the present invention includes various embodiments and the like not described here. Therefore, the technical range of the present invention is to be defined only by the inventive specific matter according to the adequate claims from the above description.

### [Industrial Applicability]

According to the characteristics of the present invention, it is possible to provide an absorbent article, such as a disposable diaper, by which it is possible to maintain the engagement force of the fastening tape even after repeated use while ensuring the strength of the outer-layer nonwoven fabric, in a case of using a structure in which rather than making use of a target tape, the fastening tape is engaged directly with the outer-layer nonwoven fabric.

### [Reference Signs List]

- 1: Disposable diaper
- 10: Topsheet
- 15: Lower-layer sheet
- 20: barrier sheet
- 30: Absorber
- 40: Outer-layer nonwoven fabric
- 45: LSG nonwoven fabric
- 50: Absorbent chassis
- 60: Side panels
- 71, 72: Fastening tapes
- 71a, 72a: Male members
- 80: Adhesive layer
- 90: Additional adhesive layer
- 81, 91, 92: Adhesive

## Claims

1. An absorbent article (1) comprising:
a substantially longitudinally-elongated absorbent chassis (50) comprising a liquid-permeable topsheet (10), a liquid-impermeable barrier sheet (20), a liquid-retentive absorber (30) provided between the topsheet and the barrier sheet, and an outer-layer nonwoven fabric (40) provided outside the barrier sheet;
side panels (60) extending from sides of the absorbent chassis;
a male member (71a, 72a) of a mechanical fastener provided at sides of the side panels; and an adhesive layer (80) arranged between the barrier sheet and the outer-layer nonwoven fabric, and formed by an adhesive (81) that bonds the barrier sheet and the outer-layer nonwoven fabric, wherein
the adhesive layer has an application pattern of the adhesive, the application pattern crossing an orientation of fibers forming the outer-layer nonwoven fabric and an orientation of a resin forming the barrier sheet,
wherein a lower-layer sheet is provided between the barrier sheet and the absorber, and
an additional adhesive layer (90) is formed by an adhesive (91, 92) that bonds the barrier sheet and the lower-layer sheet, wherein
the additional adhesive layer comprises:
a first portion having an application pattern of the adhesive, the pattern crossing the orientation of the resin configuring the barrier sheet, and
a second portion having an application pattern of the adhesive, the pattern being approximately parallel to the orientation of the resin.

2. The absorbent article according to claim 1, wherein the application pattern of the adhesive layer has spiral shape repeated along the longitudinal direction of the absorbent chassis.

3. The absorbent article according to claim 1 or 2, wherein an interval between the first portion and the second portion in a shorter direction perpendicular to the longitudinal direction of the absorbent chassis is 20 mm or less.

4. The absorbent article according to any preceding claim, wherein
the application pattern of the additional adhesive layer has spiral shape repeated along the longitudinal direction of the absorbent chassis in the first portion, and has linear shaped extending along the longitudinal direction of the absorbent chassis in the second portion.

5. The absorbent article according to any preceding claim, wherein the side panels expand and contract in a shorter direction perpendicular to the longitudinal direction of the absorbent chassis.

## Patentansprüche

1. Saugfähiger Artikel (1), umfassend:
einen im Wesentlichen in Längsrichtung länglichen saugfähigen Rahmen (50) mit einer flüssigkeitsdurchlässigen Oberlage (10), einer flüssigkeitsundurchlässigen Sperrlage (20), einem Flüssigkeit aufnehmenden Absorber (30) zwischen der Oberlage und der Sperrlage und einem Außenschicht-Faservlies (40) außerhalb der Sperrlage;
von den Seiten des saugfähigen Rahmens ausgehende Seitenteile (60);
ein Einsteckelement (71a, 72a) eines mechanischen Befestigungsmittels an den Seiten der Seitenteile; und eine Klebeschicht (80) zwische der Sperrlage und dem Außenschicht-Faservlies, die von einem Kleber (81) gebildet ist, der die Sperrlage und das Außenschicht-Faservlies miteinander verbindet, wobei
die Klebeschicht ein Kleberauftragmuster aufweist, das eine Ausrichtung der das Außenschicht-Faservlies bildenden Fasern und eine Ausrichtung eines sich in der Sperrlage bildenden Harzes überkreuzt,
wobei zwischen der Sperrlage und dem Absorber eine Unterlage vorgesehen ist, und
eine zusätzliche Klebeschicht (90) von einem Kleber (91, 92) gebildet wird, der die Sperrlage und die Unterlage miteinander verbindet, wobei
die zusätzliche Klebeschicht umfasst:
eine erste Partie mit einem Kleberauftragmuster, das die Ausrichtung des die Sperrlage bildenden Harzes überkreuzt, und
eine zweite Partie mit einem Kleberauftragmuster, das ungefähr parallel zur Ausrichtung des Harzes verläuft.

2. Saugfähiger Artikel nach Anspruch 1, wobei das Auftragmuster der Klebeschicht eine Spiralform hat, die sich entlang der Längsrichtung des saugfähigen Rahmens wiederholt.

3. Saugfähiger Artikel nach Anspruch 1 oder 2, wobei ein Intervall zwischen der ersten Partie und der zweiten Partie in einer kürzeren Richtung senkrecht zur Längsrichtung des saugfähigen Rahmens höchstens 20 mm beträgt.

4. Saugfähiger Artikel nach einem der vorhergehenden Ansprüche, wobei
wobei das Auftragmuster der zusätzlichen Klebeschicht in der ersten Partie eine Spiralform hat, die sich entlang der Längsrichtung des saugfähigen Rahmens wiederholt, und in der zweiten Partie eine Linearform hat, die sich entlang der Längsrichtung des saugfähigen Rahmens erstreckt.

5. Saugfähiger Artikel nach einem der vorhergehenden Ansprüche, wobei die Seitenteile sich in einer kürzeren Richtung senkrecht zur Längsrichtung des saugfähigen Rahmens ausdehnen und zusammenziehen.

## Revendications

1. Article absorbant (1) comportant :
une armature absorbante sensiblement allongée dans le sens longitudinal (50) comportant une feuille de dessus perméable aux liquides (10), une feuille barrière imperméable aux liquides (20), un élément absorbant de retenue de liquides (30) mis en oeuvre entre la feuille de dessus et la feuille barrière, et un tissu non tissé formant couche extérieure (40) mis en oeuvre à l'extérieur de la feuille barrière ;
des panneaux latéraux (60) s'étendant depuis des côtés de l'armature absorbante ;
un élément mâle (71a, 72a) d'une attache mécanique mise en oeuvre au niveau de côtés des panneaux latéraux ; et une couche adhésive (80) agencée entre la feuille barrière et le tissu non tissé formant couche extérieure, et formée par un adhésif (81) qui colle la feuille barrière et le tissu non tissé formant couche extérieure, dans lequel
la couche adhésive a un motif d'application de l'adhésif, le motif d'application croisant une orientation des fibres formant le tissu non tissé formant couche extérieure et une orientation d'une résine formant la feuille barrière,
dans lequel une feuille formant couche inférieure est mise en oeuvre entre la feuille barrière et l'élément absorbant, et
une couche adhésive supplémentaire (90) est formée par un adhésif (91, 92) qui colle la feuille barrière et la feuille formant couche inférieure, dans lequel
la couche adhésive supplémentaire comporte :
une première partie ayant un motif d'application de l'adhésif, le motif croisant l'orientation de la résine configurant la feuille barrière, et
une deuxième partie ayant un motif d'application de l'adhésif, le motif étant approximativement parallèle par rapport à l'orientation de la résine.

2. Article absorbant selon la revendication 1, dans lequel le motif d'application de la couche adhésive a une forme de spirale répétée le long de la direction allant dans le sens longitudinal de l'armature absorbante.

3. Article absorbant selon la revendication 1 ou la revendication 2, dans lequel un intervalle entre la première partie et la deuxième partie dans une direction plus courte perpendiculaire par rapport à la direction allant dans le sens longitudinal de l'armature absorbante mesure 20 mm ou moins.

4. Article absorbant selon l'une quelconque des revendications précédentes, dans lequel
le motif d'application de la couche adhésive supplémentaire a une forme de spirale répétée le long de la direction allant dans le sens longitudinal de l'armature absorbante dans la première partie, et a une forme linéaire s'étendant le long de la direction allant dans le sens longitudinal de l'armature absorbante dans la deuxième partie.

5. Article absorbant selon l'une quelconque des revendications précédentes, dans lequel les panneaux latéraux s'étendent et se contractent dans une direction plus courte perpendiculaire par rapport à la direction allant dans le sens longitudinal de l'armature absorbante.
